(19) Europäisches Patentamt
European Patent Office
Office européen des brevets

(11) Veröffentlichungsnummer: **0 003 519**
**A1**

(12) **EUROPÄISCHE PATENTANMELDUNG**

(21) Anmeldenummer: **79100183.7**

(22) Anmeldetag: **22.01.79**

(51) Int. Cl.²: **C 07 J 5/00,** C 07 J 7/00, C 07 J 17/00, A 61 K 31/57

(30) Priorität: **25.01.78 DE 2803660**

(43) Veröffentlichungstag der Anmeldung: **22.08.79**
**Patentblatt 79/17**

(84) Benannte Vertragsstaaten: **BE CH DE FR GB IT LU NL SE**

(71) Anmelder: **SCHERING Aktiengesellschaft Berlin und Bergkamen, Müllerstrasse 170-178, D-1000 Berlin 65 (DE)**

(72) Erfinder: **Dahl, Helmut, Dr., Gollanczstrasse 102, D-1000 Berlin 28 (DE)**
Erfinder: **Schöttle, Ernst, Dr., Neidenburger Allee 28, D-1000 Berlin 19 (DE)**
Erfinder: **Wieske, Reinhold, Dr., Am Rupenhorn 6a, D-1000 Berlin 19 (DE)**
Erfinder: **Weber, Alfred, Dr., Schützallee 56, D-1000 Berlin 37 (DE)**
Erfinder: **Kennecke, Mario, Dr., Taubertstrasse 31 f, D-1000 Berlin 33 (DE)**

(54) **Neue in 17-Position substituierte Steroide der Pregnanreihe, ihre Herstellung und Verwendung.**

(57) 1. Steroide der allgemeinen Formel I

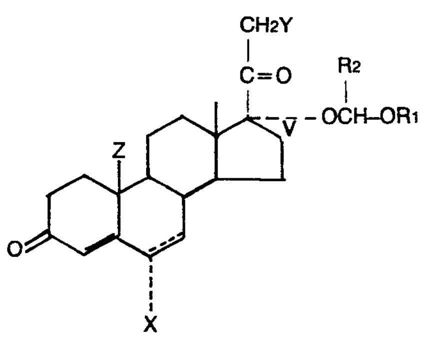

(I),

worin

..... eine Einfachbindung oder eine Doppelbindung
X ein Wasserstoffatom, ein Fluoratom oder eine Methylgruppe,
Y ein Wasserstoffatom, eine Hydroxygruppe oder eine Alkanoyloxygruppe mit 1 bis 6 Kohlenstoffatomen,
Z ein Wasserstoffatom oder eine Methylgruppe und
V eine Methylengruppe, eine Äthylidengruppe, eine Hydroxymethylengruppe oder eine Vinylidengruppe bedeuten und
R'₁ eine 1 bis 6 Kohlenstoffatome enthaltende, gegebenenfalls durch ein Sauerstoffatom unterbrochene Alkylgruppe und
R'₂ ein Wasserstoffatom oder eine 1 bis 6 Kohlenstoffatome enthaltende Alkylgruppe darstellen oder
R'₁ und R'₂ gemeinsam eine Tri- oder Tetramethylengruppe bedeuten, falls
Y eine Hydroxygruppe oder eine Alkanoyloxygruppe ist, sind Zwischenprodukte und pharmakologisch wirksame Substanzen.

EP 0 003 519 A1

Die Erfindung betrifft den in den Patentansprüchen gekennzeichneten Gegenstand.

Bekanntlich werden antiinflammatorisch wirksame 11ß-Hydroxysteroide (wie zum Beispiel die Kortikoide: Hydrocortison, Prednisolon, Dexamethason, Betamethason, Prednyliden, Triamcinolon, Fluocinolon oder Flurandrenolon) mittels einer sehr aufwendigen vielstufigen Partialsynthese aus natürlich vorkommenden Steroiden (wie Diosgenin) hergestellt, welche immer schwieriger in ausreichenden Mengen beschafft werden können. Innerhalb der vielstufigen Synthese dieser Verbindungen ist die mikrobiologische Einführung der 11ß-Hydroxygruppe in das Steroidgerüst in der Regel der aufwendigste und verlustreichste Syntheseschritt.

Im Jahre 1966 wurde ein Verfahren entwickelt, mit dessen Hilfe man die Ausbeute bei der 11ß-Hydroxylierung von 11-Desoxy-17α-Hydroxysteroiden der Pregnanreihe wesentlich steigern kann, indem man die 17α-Hydroxygruppe verestert, dann mittels Pilzen der Gattung Curvularia hydroxyliert und die erhaltenen 11ß-Hydroxy-17α-acyloxysteroide verseift (Deutsches Patent 1 618 599).

Die Acylierung der 17-Hydroxygruppe ist aber recht aufwendig, die hierbei erzielten Ausbeuten oft unbefriedigend.

Schwierig ist auch die Hydrolyse der 11ß-Hydroxy-17α-acyloxysteroide, da sich meist Nebenprodukte bilden und somit eine aufwendige und verlustreiche Aufreinigung der erhaltenen Produkte erfordert, damit diese den für Arzneimittelwirkstoffe erforderlichen Reinheitskriterien entsprechen.

Es wurde nun gefunden, daß man das Verfahren zur 11ß-Hydroxylierung von Steroiden wesentlich verbessern kann, wenn man zur mikrobiologischen Umsetzung anstelle der 11-Desoxy-17α-acyloxysteroide als Ausgangsverbindung Steroide der allgemeinen Formel I gemäß Anspruch 1 verwendet.

Unter einer Alkylgruppe $R_1$ oder $R_2$ der Steroide der allgemeinen Formel I gemäß Anspruch 1 soll beispielsweise eine 1 bis 6 Kohlenstoffatome, insbesondere 1 bis 4 Kohlenstoffatome enthaltende - vorzugsweise geradkettige - Alkylgruppe verstanden werden. Geeignete Alkylgruppen $R_1$ oder $R_2$ sind beispielsweise die Propylgruppe, die Butylgruppe, die Isopropylgruppe, die sek.-Butylgruppe und insbesondere die Methylgruppe und die Äthylgruppe. Unter einer durch ein Sauerstoffatom unterbrochenen Alkylgruppe soll vorzugsweise eine Gruppe verstanden werden, welche 3 bis 6 Kohlenstoffatome besitzt. Solche Gruppen sind beispielsweise 2-Alkoxyäthylengruppen wie die 2-Methoxy-äthylengruppe oder die 2-Äthoxy-äthylengruppe.

Die Steroide der allgemeinen Formel I gemäß Anspruch 12 können in einfacher Weise nach dem erfindungsgemäßen Verfahren gemäß Anspruch hergestellt werden.

Die Acetalisierung kann unter Bedingungen durchgeführt werden, die an sich bekannt sind (Synthesis 1975, 2786, J.Chem.Soc., (c) 1974, 431 und J.Amer.Chem.Soc. 74, 1239 (1952).

So kann man beispielsweise die Steroide der allgemeinen Formeln II oder III mit einem Acetal der allgemeinen Formel IV in Gegenwart von sauren Katalysatoren, wie zum Beispiel Perchlorsäure, p-Toluolsulfonsäure oder zweckmäßigerweise Phosphorpentoxid umsetzen. Diese Reaktion kann in Abwesenheit weiterer Lösungsmittel oder in Gegenwart von

inerten Lösungsmitteln (Chloroform, Methylenchlorid, Tetrachloräthan, Tetrachlormethan, Toluol, Diäthyläther, Tetrahydrofuran, Dioxan etc.) durchgeführt werden. Die Reaktion wird üblicherweise bei einer Reaktionstemperatur zwischen -20 °C bis +50 °C durchgeführt und eignet sich insbesondere zur Herstellung solcher Steroide der allgemeinen Formel I mit $R_2$ in der Bedeutung von Wasserstoff.

Andererseits kann man die Steroide der allgemeinen Formeln II oder III auch mit einem Vinyläther der allgemeinen Formel V umsetzen. Diese Reaktion wird vorzugsweise in einem der obengenannten inerten Lösungsmittel unter Zusatz saurer Katalysatoren (Perchlorsäure, p-Toluolsulfonsäure, Methansulfonsäure etc.) durchgeführt. Vorzugsweise erfolgt die Reaktion bei einer Reaktionstemperatur von -20 °C bis 100 °C.

Zur Erzielung optimaler Ausbeuten ist es zweckmäßig, die Reaktionsparameter in Vorversuchen wie sie dem Fachmann geläufig sind, zu variieren.

Für den Fachmann war es nicht vorhersehbar, daß sich die tertiäre und zudem sterisch gehinderte 17α-Hydroxygruppe der Steroide der allgemeinen Formeln II oder III praktisch quantitativ acetalisieren läßt.

Die sich gegebenenfalls anschließende Verseifung erfolgt unter den üblichen Bedingungen, indem man die erhaltenen Produkte in wasser- oder alkoholhaltiger Lösung mit basischen Katalysatoren (Kaliumhydrogenkarbonat, Natriumcarbonat, Kaliumkarbonat, Natriumhydroxid, Natriummethylat etc.) umsetzt.

Die sich gegebenenfalls anschließende Oxydation der 3ß-Hydroxygruppe unter gleichzeitiger Isomerisierung der $\Delta^5$-Doppelbindung erfolgt

beispielsweise unter den Bedingungen, die unter dem Namen "Oppenauer-Oxydation" wohlbekannt sind.

Besonders gut gelingt die sich gegebenenfalls anschließende Verseifung, Oxydation und Isomerisierung, wenn man die aus den Steroiden der allgemeinen Formel III erhaltenen Verbindungen mit einer Kultur der Spezies Flavobacterium dehydrogenaus umsetzt.

Die erhaltenen Verfahrensprodukte lassen sich dann unter den Bedingungen wie sie für die Herstellung von 11ß-Hydroxy-17α-acyloxysteroiden bekannt sind (Deutsches Patent 1 618 599) in der 11ß-Position hydroxylieren.

Nach der Hydroxylierung kann die 17ständige Acetalgruppe praktisch quantitativ gespalten werden.

Diese Spaltung wird unter den Bedingungen durchgeführt, welche man konventionellerweise zur Hydrolyse oder Alkoholyse von Acetalen anwendet. So kann man beispielsweise die Verbindungen spalten, indem man sie in einem niederen Alkohol wie Methanol oder Äthanol oder in einem wasserhaltigen organischen Lösungsmittel wie Glykolmonomethyläther, Tetrahydrofuran, Dioxan, Dimethylformamid, Dimethylsulfoxyd, Hexamethylphosphorsäuretriamid, Aceton mit einer Mineralsäure wie Salzsäure, Schwefelsäure, Phosphorsäure, Perchlorsäure, einer Sulfonsäure wie p-Toluolsulfonsäure, einer stark aciden Carbonsäure wie Ameisensäure, Essigsäure, Trifluoressigsäure, saure Ionenaustauscher oder mit einer Lewis Säure wie Bortrifluorid, Zinkchlorid, Zinkbromid oder Titantetrachlorid umsetzt.

Die neuen Verbindungen der allgemeinen Formel I gemäß Anspruch 1 sind aber nicht nur wertvolle Zwischenprodukte sondern besitzen

darüber hinaus eine gute pharmakologische Wirksamkeit, die oft diejenige der 17α-Hydroxyverbindungen der allgemeinen Formel II gemäß Anspruch 12 beziehungsweise deren Ester übertrifft, und zeichnen sich oft durch eine günstige Dissoziation zwischen erwünschter Wirksamkeit und unerwünschten Nebenwirkungen aus.

Die nachfolgenden Beispiele dienen zur Erläuterung der Erfindung.

Beispiel 1

a) 21,63 g 3ß,21-Diacetoxy-17α-hydroxy-5-pregnen-20-on werden in 150 ml wasserfreiem Methylenchlorid und 100 ml wasserfreiem Formaldehyddimethylacetal gelöst. Dann kühlt man die Lösung mit Wasser und trägt in sie ein Gemisch von 21,6 g Phosphorpentoxyd und 43 g Kieselgur ein und rührt eine Stunde lang bei Raumtemperatur. Dann filtriert man die Reaktionsmischung und wäscht den Rückstand mit Methylenchlorid, setzt dem Filtrat Triäthylamin zu, bis ein pH-Wert von 9 erreicht wird und engt es im Vakuum ein. Der Rückstand wird aus Methanol-Methylenchlorid umkristallisiert und man erhält 22,68 g 3ß,21-Diacetoxy-17α-methoxymethoxy-5-pregnen-20-on vom Schmelzpunkt 182-184 °C.

b) Ein 2 l Erlenmeyerkolben mit 1 Liter steriler Nährlösung enthaltend 0,3 % Hefeextrakt, 0,3 % Cornsteep liquor und 0,2 % Stärkezucker - eingestellt auf pH = 7,0 - wird mit einer Trockenkultur von Flavobacterium dehydrogenans ATCC 13 930 beimpft und bei 30 °C zwei Tage lang mit 175 Umdrehungen pro Minute geschüttelt.

Ein 500 ml Erlenmeyerkolben mit 85 ml des gleichen Nährmediums wird mit 10 ml der Flavobacterium dehydrogenans Anzuchtkultur beimpft und bei 30 °C 7 Stunden lang mit 175 Umdrehungen pro Minute geschüttelt. Dann setzt man der Kultur 5 ml einer sterilen Lösung von 0,5 g 3ß,21-Diacetoxy-17α-methoxymethoxy-5-pregnen-20-on in Dimethylformamid zu und schüttelt bei 30 °C weitere 65 Stunden

lang mit 175 Umdrehungen pro Minute. Nach erfolgter Fermentation wird die Kultur zweimal mit 100 ml Äthylenchlorid extrahiert, der Extrakt im Vakuum eingeengt, der Rückstand durch Chromatographie über Aluminiumoxid gereinigt und man erhält 402 mg 21-Hydroxy-17α-methoxymethoxy-4-pregnen-3,20-dion vom Schmelzpunkt 152 - 153 °C.

c) Ein 2 l Erlenmeyerkolben mit 1 Liter steriler Nährlösung enthaltend 2 % Glukose und 2 % Cornsteep liquor - eingestellt auf pH = 6,5 - wird mit einer Abschwemmung einer Trockenkultur von Curvularia lunata NRRL 2380 beimpft und bei 30 °C 60 Stunden lang mit 175 Umdrehungen pro Minute geschüttelt.

Ein 500 ml Erlenmeyerkolben mit 90 ml eines sterilen Nährmediums enthaltend 1,0 % Cornsteep liquor und 1,25 % Sojapuder - eingestellt auf pH = 6,2 - wird mit 10 ml der Curvularia lunata Anzuchtskultur beimpft und bei 30 °C 7 Stunden lang mit 175 Umdrehungen pro Minute geschüttelt. Dann setzt man der Kultur 0,6 ml einer sterilen Lösung von 30 mg 21-Hydroxy-17α-methoxymethoxy-4-pregnen-3,20-dion in Dimethylformamid zu und fermentiert weitere 65 Stunden unter den angegebenen Bedingungen.

Man arbeitet die Fermentationskultur auf, wie im Beispiel 1b beschrieben und erhält 27 mg 11ß,21-Dihydroxy-17α-methoxymethoxy-4-pregnen-3,20-dion vom Schmelzpunkt 180 - 182 °C.

d) 2,5 g 11ß,21-Dihydroxy-17α-methoxymethoxy-4-pregnen-3,20-dion werden in 40 ml wasserfreiem Methylenchlorid gelöst, auf 0 °C gekühlt und innerhalb von 15 Minuten unter Argon mit einer Lösung von 2,25 ml Titantetrachlorid in 10 ml Methylenchlorid versetzt. Man läßt die Reaktionsmischung 90 Minuten lang bei Raumtemperatur rühren, setzt dann 150 ml Methylenchlorid und 100 ml gesättigte wässrige Natriumhydrogenkarbonatlösung zu, wäscht sie neutral, trocknet sie über Natriumsulfat und engt sie im Vakuum ein. Der Rückstand wird aus Chloroform umkristallisiert und man erhält 2,19 g 11ß,17α,21-Trihydroxy-4-pregnen-3,20-dion vom Zersetzungspunkt 215 °C.

Beispiel 2

a) 50 g 3ß,21-Diacetoxy-17α-hydroxy-5-pregnen-20-on werden mit 50 mg wasserfreier p-Toluolsulfonsäure und 350 ml wasserfreiem Methylenchlorid versetzt, auf 0 °C gekühlt und nach Zugabe von 10 g Methylvinyläther 4 Stunden lang bei 0 °C gerührt. Dann setzt man der Reaktion Triäthylamin zu, bis ein pH-Wert von 9 erreicht ist und engt sie im Vakuum ein. Man erhält 58 g 3ß,21-Diacetoxy-17α-(1'-methoxyäthoxy)-5-pregnen-20-on als Diastereomerengemisch vom Schmelzpunkt 80 - 118 °C (Eine aus Methanol umkristallisierte Probe schmilzt bei 132 - 134 °C).

b) Unter den Bedingungen des Beispiels 1b wird eine Lösung von 600 mg 3ß,21-Diacetoxy-17α-(1'-methoxyäthoxy)-5-pregnen-20-on Diastereomerengemisch in 5 ml Dimethylformamid

mit einer Flavobacterium dehydrogenans ATCC 13930 Kultur umgesetzt, aufbereitet und man erhält 394 mg 21-Hydroxy-17α-(1'-methoxyäthoxy)-4-pregnen-3,20-dion als Diastereomerengemisch vom Schmelzpunkt 166 - 178 °C.

c) Eine Lösung von 100 mg 21-Hydroxy-17α-(1'-methoxyäthoxy)-4-pregnen-3,20-dion Diastereomerengemisch in 2 ml Dimethylformamid wird unter den im Beispiel 1c beschriebenen Bedingungen mit einer Kultur von Curvularia lunata NRRL 2380 fermentiert, aufbereitet und man erhält 105 mg 11ß,21-Dihydroxy-17α-(1'-methoxyäthoxy)-4-pregnen-3,20-dion als öliges Diastereomerengemisch.

Dieses Gemisch wird mit 3 ml Methanol und 0,5 ml 2n wässriger Salzsäure versetzt und 5 Stunden lang bei Raumtemperatur geschüttelt. Anschließend setzt man der Mischung 4 ml Wasser zu, neutralisiert sie mit gesättigter wässriger Natriumhydrogenkarbonat-Lösung, extrahiert zweimal mit je 8 ml Äthylenchlorid, engt die organische Phase im Vakuum ein, reinigt den Rückstand durch Chromatographie über eine Aluminiumoxid-Säule und erhält 69 mg 11ß,17α,21-Trihydroxy-4-pregnen-3,20-dion vom Schmelzpunkt 217 - 219 °C.

<u>Beispiel 3</u>

a) 10,0 g 21-Acetoxy-17α-hydroxy-4-pregnen-3,20-dion werden mit 13 mg wasserfreier p-Toluolsulfonsäure und 130 ml wasserfreiem Methylenchlorid versetzt, auf 0 °C gekühlt und nach Zugabe von 2,3 g Methylvinyläther 7 Stunden lang bei 0 °C gerührt. Man arbeitet die Reaktionsmischung auf, wie im Beispiel 2a beschrieben und erhält

11,6 g 21-Acetoxy-17α-(1'-methoxyäthoxy)-4-pregnen-3,20-dion vom Schmelzpunkt 135 - 150 °C.

b) Eine Lösung von 0,1 g 21-Acetoxy-17α-(1'-methoxyäthoxy)-4-pregnen-3,20-dion Diastereomerengemisch in 2 ml Dimethylformamid wird unter den im Beispiel 1c beschriebenen Bedingungen mit einer Kultur von Curvularia lunata NRRL 2380 fermentiert und aufbereitet. Das so erhaltene 11ß,21-Dihydroxy-17α-(1'-methoxyäthoxy)-4-pregnen-3,20-dion wird dann unter den im Beispiel 2c beschriebenen Bedingungen hydrolysiert und man erhält 78 mg 11ß,17α,21-Trihydroxy-4-pregnen-3,20-dion vom Schmelzpunkt 217 - 219 °C.

Beispiel 4

a) 10,0 g 21-Acetoxy-17α-hydroxy-4-pregnen-3,20-dion werden unter den im Beispiel 3a beschriebenen Bedingungen mit 2,50 g Äthylvinyläther umgesetzt, aufbereitet und man erhält 12,5 g 21-Acetoxy-17α-(1'-äthoxyäthoxy)-4-pregnen-3,20-dion als öliges Diastereomerengemisch.

b) Eine Lösung von 0,1 g 21-Acetoxy-17α-(1'-äthoxyäthoxy)-4-pregnen-3,20-dion Gemisch in 2 ml Dimethylformamid wird unter den im Beispiel 1c beschriebenen Bedingungen mit Curvularia lunata NRRL 2380 hydroxyliert aufbereitet und man erhält das 17α-(1'-Äthoxyäthoxy)-11ß,21-dihydroxy-4-pregnen-3,20-dion, welches unter den im Beispiel 2c beschriebenen Bedingungen zu 63 mg 11ß,17α,21-Trihydroxy-4-pregnen-3,20-dion vom Schmelzpunkt 216 - 217,5 °C hydrolysiert wird.

Beispiel 5

a) 10,0 g 21-Acetoxy-17α-hydroxy-4-pregnen-3,20-dion werden unter den im Beispiel 3a beschriebenen Bedingungen mit 4,0 g Isobutylvinyläther umgesetzt, aufbereitet und man erhält 13,5 g 21-Acetoxy-17α-(1'-isobutyloxyäthoxy)-4-pregnen-3,20-dion als öliges Diastereomerengemisch.

b) Eine Lösung von 0,1 g 21-Acetoxy-17α-(1'-isobutyloxyäthoxy)-4-pregnen-3,20-dion Gemisch in 2 ml Dimethylformamid wird unter den im Beispiel 1c beschriebenen Bedingungen mit Curvularia lunata NRRL 2380 hydroxyliert, aufbereitet und man erhält das 11ß,21-Dihydroxy-17α-(1'isobutyloxyäthoxy)-4-pregnen-3,20-dion, welches unter den im Beispiel 2c beschriebenen Bedingungen zu 68 mg 11ß,17α,21-Trihydroxy-4-pregnen-3,20-dion vom Schmelzpunkt 214 - 216 °C (Zersetzung) verseift wird.

Beispiel 6

a) 1,95 g 21-Acetoxy-17α-hydroxy-4-pregnen-3,20-dion werden mit 5 mg wasserfreier p-Toluolsulfonsäure, 25 ml wasserfreiem Methylenchlorid und 3,5 ml Dihydropyran versetzt und 13 Stunden lang bei Raumtemperatur gerührt. Man arbeitet die Reaktionsmischung auf, wie im Beispiel 3a beschrieben und erhält 2,0 g 21-Acetoxy-17α-(2'-tetrahydropyranyloxy)-4-pregnen-3,20-dion als Diastereomerengemisch vom Schmelzpunkt 185 - 200 °C.

b) Eine Lösung von 0,1 g 21-Acetoxy-17α-(2'-tetrahydropyranyloxy)-4-pregnen-3,20-dion Gemisch in 2 ml Dimethylformamid wird unter den Bedingungen, wie im Beispiel 1c beschrieben, mit Curvularia lunata NRRL 2380 hydroxyliert, aufbereitet und man erhält das 11ß,21-Dihydroxy-17α-(2'-tetrahydropyranyloxy)-4-pregnen-3,20-dion, welches unter den im Beispiel 2 c beschriebenen Bedingungen zu 72 mg 11ß,17α,21-Trihydroxy-4-pregnen-3,20-dion vom Schmelzpunkt 215 °C (Zersetzung) hydrolysiert wird.

<u>Beispiel 7</u>

a) 50 g 21-Acetoxy-17α-hydroxy-6α-methyl-4-pregnen-3,20-dion werden unter den im Beispiel 3a beschriebenen Bedingungen mit 13,9 g Methylvinyläther umgesetzt, aufbereitet und man erhält 59 g 21-Acetoxy-17α-(1'-methoxyäthoxy)-6α-methyl-4-pregnen-3,20-dion als amorphe Masse.

b) Eine Lösung von 200 mg 21-Acetoxy-17α-(1'-methoxyäthoxy)-6α-methyl-4-pregnen-3,20-dion in 0,4 ml Dimethylformamid wird unter den im Beispiel 1c beschriebenen Bedingungen mit Curvularia Lunata NRRL 2380 hydroxyliert, aufbereitet und man erhält das 11ß,21-Dihydroxy-17α-(1'-methoxyäthoxy)-6α-methyl-4-pregnen-3,20-dion, welches unter den im Beispiel 2c beschriebenen Bedingungen zu 15 mg 11ß,17α,21-Trihydroxy-4-pregnen-3,20-dion vom Schmelzpunkt 189 - 192 °C hydrolysiert wird.

Beispiel 8

a) 2,0 g 21-Acetoxy-17α-hydroxy-16ß-methyl-4-pregnen-3,20-dion werden mit 5 mg p-Toluolsulfonsäure (wasserfrei) und 25 ml wasserfreiem Methylenchlorid versetzt und auf 0 °C gekühlt. Dann setzt man der Mischung unter Rühren 0,5 g Methylvinyläther zu, rührt 5 Stunden lang bei 0 °C und weitere 12 Stunden bei Raumtemperatur, arbeitet die Reaktionsmischung auf, wie im Beispiel 3a beschrieben und erhält 2,1 g 21-Acetoxy-17α-(1'-methoxyäthoxy)-16ß-methyl-4-pregnen-3,20-dion als öliges Diastereomerengemisch.

b) Eine Lösung von 50 mg 21-Acetoxy-17α-(1'-methoxyäthoxy)-16ß-methyl-4-pregnen-3,20-dion Gemisch in 1 ml Dimethylformamid wird unter den im Beispiel 1c beschriebenen Bedingungen mit Curvularia lunata NRRL 2380 hydroxyliert, aufbereitet und man erhält das 11ß,21-Dihydroxy-17α-(1'-methoxyäthoxy)-16ß-methyl-4-pregnen-3,20-dion, welches unter den im Beispiel 2c beschriebenen Bedingungen zu 32 mg 11ß,17α,21-Trihydroxy-16ß-methyl-4-pregnen-3.20-dion vom Schmelzpunkt 204-207 °C hydrolysiert wird.

Beispiel 9

a) 50 g 17α-Hydroxy-4-pregnen-3,20-dion werden unter den im Beispiel 3α beschriebenen Bedingungen mit 15 g Methylvinyläther umgesetzt, aufbereitet und man erhält 51,2 g 17α-(1'-Methoxyäthoxy)-4-pregnen-3,20-dion vom Schmelzpunkt 115 - 152 °C.

b) Eine Lösung von 0,1 g 17α-(1'-Methoxyäthoxy)-4-pregnen-3,20-dion in 1 ml Dimethylformamid wird unter den im Beispiel 1c beschriebenen Bedingungen mit einer Kultur von Curvularia lunata NRRL 2380 fermentiert und aufbereitet. Das so erhaltene 11ß-Hydroxy-17α-(1'-methoxyäthoxy)-4-pregnen-3,20-dion (Schmelzpunkt 85 - 103 °C) wird unter den im Beispiel 2c beschriebenen Bedingungen hydrolysiert und man erhält 63 mg 11ß,17α-Dihydroxy-4-pregnen-3,20-dion vom Schmelzpunkt 222 - 223,5 °C.

Beispiel 10

a) 50 g 3ß,21-Diacetoxy-17α-hydroxy-5-pregnen-20-on werden unter den im Beispiel 1a beschriebenen Bedingungen in 150 ml Methylenchlorid mit 380 g Formaldehyd-bisglykolmonomethylätheracetal, 50 g Phosphorpentoxid und 100 g Kieselgur umgesetzt, aufbereitet und man erhält 45,8 g 3ß,21-Diacetoxy-17α-(2'-methoxyäthoxy-methoxy)-5-pregnen-20-on vom Schmelzpunkt 160 - 161 °C.

b) Unter den im Beispiel 1b beschriebenen Bedingungen werden 0,5 g 3ß,21-Diacetoxy-17α-(2'-methoxyäthoxy-methoxy)-5-pregnen-20-on mit einer Kultur von Flavobacterium dehydrogenans ATCC 13930 umgesetzt, aufbereitet und man erhält 390 mg 21-Hydroxy-17α-(2'-methoxyäthoxy-methoxy)-4-pregnen-3,20-dion als glasige Masse.

c) Unter den Bedingungen des Beispiels 1c werden 30 mg 21-Hydroxy-17α-(2'-methoxyäthoxy-methoxy)-4-pregnen-3,20-dion mit einer Kultur von Curvularia lunata NRRL 2380 umgesetzt, aufbereitet und man erhält 24 mg 11ß,21-Dihydroxy-17α-(2'-methoxyäthoxy-methoxy)-4-pregnen-3,20-dion vom Schmelzpunkt 143 - 147 °C.

d) Unter den Bedingungen des Beispiels 1d werden 10 mg 21-Dihydroxy-17α-(2'-methoxyäthoxy-methoxy)-4-pregnen-3,20-dion in 2 ml Methylenchlorid und 0,01 ml Titantetrachlorid umgesetzt, aufbereitet und man erhält 8 mg 11ß,17α,21-Trihydroxy-4-pregnen-3,20-dion vom Zersetzungspunkt 213 °C.

Beispiel 11

20 g 3ß-Acetoxy-17α-hydroxy-5-pregnen-20-on werden unter den im Beispiel 1a bis c genannten Bedingungen umgesetzt, und man erhält 15 g 17α-Methoxymethoxy-4-pregnen-3,20-dion vom Schmelzpunkt 116 - 119 °C.

Beispiel 12

5 g 17α-Hydroxy-19-nor-4-pregnen-3,20-dion werden unter im Beispiel 1a genannten Bedingungen mit Formaldehyddimethylacetal umgesetzt, aufbereitet und man erhält das

17α-Methoxymethoxy-19-nor-4-pregnen-3,20-dion vom Schmelzpunkt 120 °C.

Beispiel 13

5 g 17α-Hydroxy-19-nor-4-pregnen-3,20-dion werden unter den im Beispiel 1a beschriebenen Bedingungen mit Formaldehyddiäthylacetal umgesetzt, aufbereitet und man erhält das 17α-Äthoxymethoxy-19-nor-4-pregnen-3,20-dion vom Schmelzpunkt 69 - 71 °C.

Beispiel 14

5 g 17α-Hydroxy-19-nor-4-pregnen-3,20-dion werden unter den im Beispiel 1a beschriebenen Bedingungen mit Formaldehyddipropylacetal umgesetzt, aufbereitet und man erhält das 17α-propoxymethoxy-19-nor-4-pregnen-3,20-dion vom Schmelzpunkt 97 - 99 °C.

Beispiel 15

50 g 21-Acetoxy-17α-hydroxy-4-pregnen-3,20-dion werden mit 600 ml Formaldehyddiäthylacetal und 600 ml Methylenchlorid suspendiert und auf -30 bis -40 °C gekühlt. Unter Rühren wird eine Mischung von 75 g Phosphorpentoxid und 150 g Kieselgur eingetragen und 30 Stunden bei -30 °C gerührt. Die Lösung wird filtriert und mit Triäthylamin neutralisiert. Nach dem Abdestillieren der Lösungsmittel wird nochmals mit Methanol abdestilliert und der Rückstand aus Methanol umkristallisiert. Man erhält 35,9 g 21-Acetoxy-17α-äthoxymethoxy-4-pregnen-3,20-dion, das nach nochmaliger Umkristallisation bei 137 - 139 °C schmilzt.

Beispiel 16

25 g 21-Acetoxy-17α-hydroxy-4-pregnen-3,20-dion werden mit 200 ml Formaldehyddipropylacetal und 320 ml Methylenchlorid suspendiert und auf -20 °C gekühlt. Unter Rühren wird eine Mischung aus 49,3 g Phosphorpentoxid und 97 g Kieselgur eingetragen und 22 Stunden bei -20 °C gerührt. Die Lösung wird filtriert und mit Triäthylamin neutralisiert. Das Methylenchlorid wird im Vakuum abdestilliert und die Formaldehyddipropylacetal-Phase vom abgeschiedenen Öl abgegossen. Nach dem Abdestillieren weiteren Lösungsmittels im Vakuum kristallisieren 19 g 21-Acetoxy-17α-propoxymethoxy-4-pregnen-3,20-dion vom Schmelzpunkt 145 - 147 °C.

Beispiel 17

50 g 21-Acetoxy-17α-hydroxy-4-pregnen-3,20-dion werden mit 500 ml Formaldehyddibutylacetal und 500 ml Methylenchlorid suspendiert und auf -35 °C gekühlt. Unter Rühren wird eine Mischung aus 74 g Phosphorpentoxid und 150 g Kieselgur eingetragen und 30 Stunden bei -35 °C gerührt. Die Lösung wird filtriert und mit Triäthylamin neutralisiert. Das Methylenchlorid wird im Vakuum abdestilliert und die Formaldehyddibutylacetal-Phase von dem abgeschiedenen Öl abgegossen. Nach dem Abdestillieren weiteren Lösungsmittels im Vakuum kristallisieren 38,7 g 21-Acetoxy-17α-butoxymethoxy-4-pregnen-3,20-dion vom Schmelzpunkt 123,5 - 124,5 °C.

Beispiel 18

10,60 g 21-Acetoxy-6α-fluor-17α-hydroxy-4-pregnen-3,20-dion werden in 265 ml Methylenchlorid und 47,7 ml Formaldehyddimethylacetal gelöst. Ein Gemisch aus 7,95 g Phosphorpentoxid und 15,0 g Kieselgur wird portionsweise zugegeben und die Mischung 90 Minuten unter Stickstoff bei Raumtemperatur gerührt. Die Lösung wird filtriert und mit 2,1 ml Triäthylamin versetzt. Die Lösungsmittel werden abdestilliert und der Rückstand aus Methanol umkristallisiert. Man erhält 7,6 g 21-Acetoxy-6α-fluor-17α-methoxymethoxy-4-pregnen-3,20-dion vom Schmelzpunkt 161 - 167 °C.

Beispiel 19

a) 43 g 3ß,21-Diacetoxy-17α-hydroxy-16ß-methyl-5-pregnen-20-on werden in 800 ml Formaldehyddimethylacetal gelöst und auf -15 °C gekühlt. In Portionen wird eine Mischung von 43 g Phosphorpentoxid und 86 g Kieselgur eingetragen und die Mischung 15 Stunden bei ca. -15 °C gerührt. Die Lösung wird filtriert, mit Triäthylamin neutralisiert und die Lösungsmittel im Vakuum abdestilliert. Der Rückstand wird mit Methanol umkristallisiert, man erhält 31,5 g 3ß,21-Diacetoxy-17α-methoxymethoxy-16ß-methyl-5-pregnen-20-on vom Schmelzpunkt 117 - 118 °C.

b) Flavobacterium dehydrogenans ATCC 13930 wird wie in Beispiel 1b, angezüchtet und fermentiert. Zur 7. Stunde setzt man der Kultur 4 ml einer sterilen Lösung von 0,2 g 3ß,21-Diacetoxy-17α-methoxymethoxy-16ß-methyl-5-pregnen-20-on in Dimethylformamid zu und schüttelt weitere 65 Stunden.
Nach erfolgter Fermentation wird die Kultur wie unter Beispiel 1b beschrieben, aufgearbeitet und man erhält 163 mg 21-Hydroxy-17α-methoxymethoxy-16ß-methyl-4-pregnen-3,20-dion vom Schmelzpunkt 126/128 - 129 °C.

Beispiel 20

29,1 g 21-Acetoxy-6-chlor-17α-hydroxy-4,6-pregnadien-3,20-dion werden in 730 ml Methylenchlorid und 131,0 ml Formaldehyddimethylacetal gelöst. Ein Gemisch aus 22,12 g Phosphorpentoxid und 44 g Kieselgur wird portionsweise zugegeben und die Mischung 2,5 Stunden unter Stickstoff bei Raumtemperatur gerührt. Die Lösung wird filtriert und mit 5,8 ml Triäthylamin versetzt. Die Lösungsmittel werden abdestilliert und der Rückstand aus Methanol unter Zusatz von Aktivkohle und 1 % Triäthylamin umkristallisiert. Man erhält 15,6 g 21-Acetoxy-6-chlor-17α-methoxymethoxy-4,6-pregnadien-3,20-dion vom Schmelzpunkt 183 - 186 °C.

Beispiel 21

a) 38,85 g 21-Acetoxy-17α-hydroxy-4-pregnen-3,20-dion werden mit 235 ml Formaldehyddiisopropylacetal und 500 ml Methylenchlorid verrührt und auf -20 °C gekühlt. Unter Rühren wird eine Mischung aus 75 g Phosphorpentoxid und 150 g Kieselgur eingetragen und 20 Stunden bei -20 °C gerührt. Die Mischung wird filtriert, mit Methylenchlorid gewaschen und mit Triäthylamin auf pH 9 gebracht. Die Lösungsmittel werden im Vakuum abdestilliert und der Rückstand in Methylenchlorid aufgenommen. Die Lösung wird mit halbgesättigter Natriumchloridlösung gewaschen, mit Natriumsulfat getrocknet, mit Aktivkohle behandelt, über Kieselgur abgesaugt und im Vakuum konzentriert. Der Rückstand wird an Kieselgel mit Toluol-Essigester-Gemischen chromatographiert. Man erhält 35,8 g 21-Acetoxy-17α-isopropoxymethoxy-4-pregnen-3,20-dion, das nach Kristallisation mit Pentan einen Schmelzpunkt von 111 - 117 °C zeigt.

## Patentansprüche

1. Steroide der allgemeinen Formel I

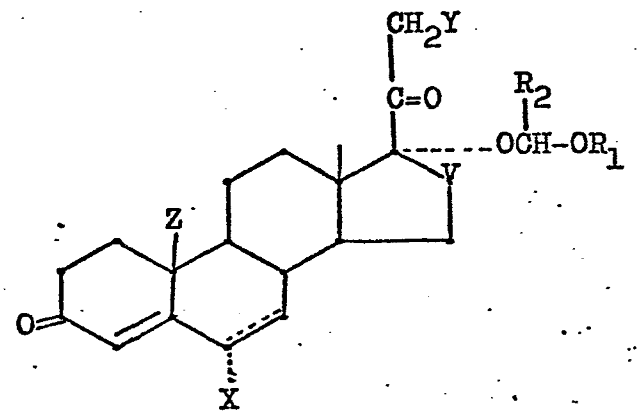

(I),

worin

..... eine Einfachbindung oder eine Doppelbindung

X ein Wasserstoffatom, ein Fluoratom oder eine Methylgruppe,

Y ein Wasserstoffatom, eine Hydroxygruppe oder eine Alkanoyloxygruppe mit 1 bis 6 Kohlenstoffatomen,

Z ein Wasserstoffatom oder eine Methylgruppe und

V eine Methylengruppe, eine Äthylidengruppe, eine Hydroxymethylengruppe oder eine Vinylidengruppe bedeuten und

$R'_1$ eine 1 bis 6 Kohlenstoffatome enthaltende, gegebenenfalls durch ein Sauerstoffatom unterbrochene Alkylgruppe und

$R'_2$ ein Wasserstoffatom oder eine 1 bis 6 Kohlenstoffatome enthaltende Alkylgruppe darstellen oder

$R'_1$ und $R'_2$ gemeinsam eine Tri- oder Tetramethylengruppe bedeuten, falls

Y eine Hydroxygruppe oder eine Alkanoyloxygruppe ist.

2. 21-Hydroxy-17α-methoxymethoxy-4-pregnen-3,20-dion.

3. 21-Acetoxy-17α-(1'-methoxyäthoxy)-4-pregnen-3,20-dion.

4. 21-Acetoxy-17α-(1'-isobutyloxyäthoxy)-4-pregnen-3,20-dion.

5. 17α-(1'-Äthoxyäthoxy)-21-acetoxy-4-pregnen-3,20-dion.

6. 21-Acetoxy-17α-(2'-tetrahydropyranyloxy)-4-pregnen-3,20-dion.

7. 21-Acetoxy-17α-(1'-methoxyäthoxy)-6α-methyl-4-pregnen-3,20-dion.

8. 21-Acetoxy-17α-(1'-methoxyäthoxy)-16ß-methyl-4-pregnen-3,20-dion.

9. 17α-(1'-Methoxyäthoxy)-4-pregnen-3,20-dion.

10. 21-Hydroxy-17α-(2'-methoxyäthoxy-methoxy)-4-pregnen-3,20-dion.

11. 17α-Methoxymethoxy-4-pregnen-3,20-dion.

12. Verfahren zur Herstellung von Steroiden der allgemeinen Formel I gemäß Anspruch 1, dadurch gekennzeichnet, daß man ein Steroid der allgemeinen Formel II oder III

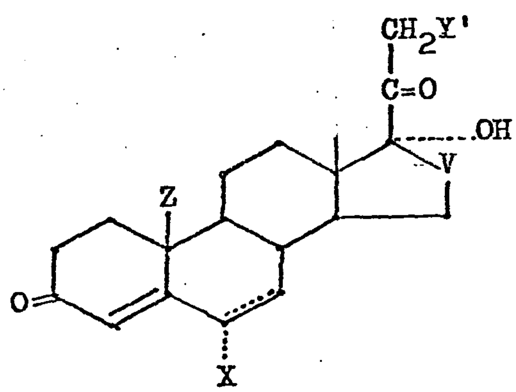

(II),

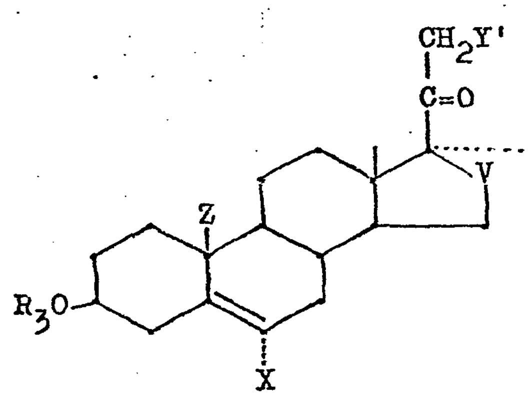

(III),

worin

.....,X, V und Z die in Anspruch 1 genannte Bedeutung besitzen und

Y' ein Wasserstoffatom oder eine Alkanoyloxygruppe mit 1 bis 6 Kohlenstoffatomen sowie

$R_3$ eine Alkanoylgruppe mit 1 bis 6 Kohlenstoffatomen darstellen,

mit einem Acetal der allgemeinen Formel IV oder einem Vinyläther der allgemeinen Formel V,

$R_2CH(OR_1)_2$ (IV) $R_4CH{=}CH{-}OR_1$ (V)

worin $R_1$ und $R_2$ die in Anspruch 1 genannte Bedeutung besitzen und $R_4$ Wasserstoff oder eine 1 bis 5 Kohlenstoffatome enthaltende Alkylgruppe oder gemeinsam mit $R_1$ eine Trimethylen- oder Äthylengruppe darstellen, falls Y eine Hydroxylgruppe oder eine Alkanoylgruppe ist, umsetzt,
gegebenenfalls Estergruppen verseift und eine 3ß-Hydroxygruppe unter gleichzeitiger Isomerisierung der $\Delta^5$-Doppelbindung oxydiert.

13. Verwendung der Steroide der allgemeinen Formel I gemäß Anspruch 1 zur Herstellung von Steroiden der allgemeinen Formel VI

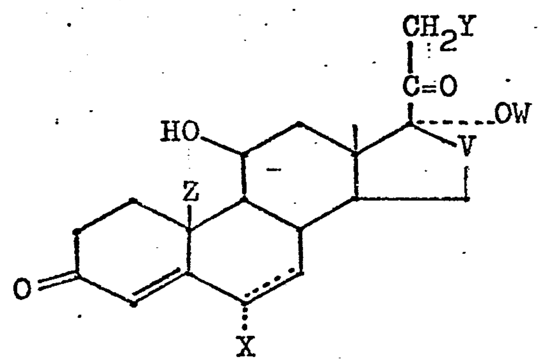

(VI),

worin W ein Wasserstoffatom oder die Gruppierung $-\overset{R_2}{\overset{|}{C}}H-OR_1$ bedeutet und .....,X,Y,Z,V,$R_1$ und $R_2$ die in Anspruch 1 genannte Bedeutung besitzen.



139-3

14. Pharmazeutische Präparate enthaltend ein oder mehrere Steroide gemäß Anspruch 1 bis 11 und 15 bis 24

15. 17α-Methoxymethoxy-19-nor-4-pregnen-3,20-dion

16. 17α-Äthoxymethoxy-19-nor-4-pregnen-3,20-dion

17. 17α-Propoxymethoxy-19-nor-4-pregnen-3,20-dion

18. 21-Acetoxy-17α-äthoxymethoxy-4-pregnen-3,20-dion

19. 21-Acetoxy-17α-propoxymethoxy-4-pregnen-3,20-dion

20. 21-Acetoxy-17α-butoxymethoxy-4-pregnen-3,20-dion

21. 21-Acetoxy-6α-fluor-17α-methoxymethoxy-4-pregnen-3,20-dion

22. 21-Hydroxy-17α-methoxymethoxy-16ß-methyl-4-pregnen-3,20-dion

23. 21-Acethoxy-6-chlor-17α-methoxymethoxy-4,6-pregnadien-3,20-dion

24. 21-Acetoxy-17α-isopropoxymethoxy-4-pregnen-3,20-dion

Europäisches Patentamt

# EUROPÄISCHER RECHERCHENBERICHT

Nummer der Anmeldung
EP 79 10 0183

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 2 924 597 (PIETRO DE RUGGIERI)<br>* Beispiel 3; Ansprüche 3,4 *<br>-- | 1,12 |
| D | DE - A - 1 618 599 (KONINKLIJKE NEDERLANDSCHE GIST-EN SPIRITUS-FABRIEK NV)<br>* Anspruch 1; Seite 5 *<br>---- | 1,13 |

KLASSIFIKATION DER ANMELDUNG (Int. Cl.)

C 07 J 5/00
7/00
17/00
A 61 K 31/57

RECHERCHIERTE SACHGEBIETE (Int. Cl.)

C 07 J 5/00
7/00
17/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 04-05-1979 | HENRY |